(19) Europäisches Patentamt
European Patent Office
Office européen des brevets

(11) Veröffentlichungsnummer: **0 419 917 B1**

(12) **EUROPÄISCHE PATENTSCHRIFT**

(45) Veröffentlichungstag der Patentschrift: **17.11.94**

(21) Anmeldenummer: **90117343.5**

(22) Anmeldetag: **08.09.90**

(51) Int. Cl.⁵: **C07D 231/14**, A01N 43/56, C07D 401/04, C07D 413/04, C07D 405/04, C07D 409/04, C07D 403/12, C07D 401/12, C07D 405/12, C07D 409/12, C07D 401/14

(54) **Pyrazol-3-carbonsäureamide.**

(30) Priorität: **23.09.89 DE 3931786**

(43) Veröffentlichungstag der Anmeldung:
**03.04.91 Patentblatt 91/14**

(45) Bekanntmachung des Hinweises auf die Patenterteilung:
**17.11.94 Patentblatt 94/46**

(84) Benannte Vertragsstaaten:
**BE CH DE FR GB IT LI NL**

(56) Entgegenhaltungen:
**EP-A- 0 177 242**
**EP-A- 0 205 023**
**EP-A- 0 244 075**
**EP-A- 0 286 279**
**EP-A- 0 322 126**
**EP-A- 0 350 176**
**DE-A- 3 332 633**

(73) Patentinhaber: **BASF Aktiengesellschaft**
**Carl-Bosch-Strasse 38**
**D-67063 Ludwigshafen (DE)**

(72) Erfinder: **Ditrich, Klaus, Dr.**
**Paray-le-Monial-Strasse 12**
**D-6702 Bad Duerkheim (DE)**
Erfinder: **Hamprecht, Gerhard, Dr.**
**Rote-Turm-Strasse 28**
**D-6940 Weinheim (DE)**
Erfinder: **Plath, Peter, Dr.**
**Hans-Balcke-Strasse 13**
**D-6710 Frankenthal (DE)**
Erfinder: **Wuerzer, Bruno, Dr.**
**Ruedigerstrasse 13**
**D-6701 Otterstadt (DE)**
Erfinder: **Westphalen, Karl-Otto, Dr.**
**Mausbergweg 58**
**D-6720 Speyer (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 419 917 B1

## Beschreibung

Die vorliegende Erfindung betrifft Pyrazol-3-carbonsäureamide der allgemeinen Formel I

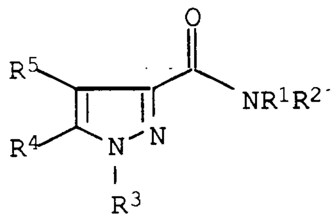

I,

$R^1$ und $R^4$
Wasserstoff;
$R^2$
$C_1$-$C_4$-Alkyl oder $C_3$-$C_8$-Cycloalkyl;
$R^3$
Wasserstoff;
$C_1$-$C_4$-Alkyl;
Phenyl, welches ein bis drei der folgenden Reste tragen kann: Halogen, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
$R^5$
eine Gruppe $COYR^6$;
Y
Sauerstoff;
$R^6$
Wasserstoff, oder eine Gruppe $-N=CR^7R^8$, wobei
$R^7$
Wasserstoff oder $C_1$-$C_4$-Alkyl und
$R^8$
$C_3$-$C_6$-Cycloalkyl oder einen Rest $R^7$ bedeutet oder
$R^7$, $R^8$
gemeinsam eine 4- bis 7-gliedrige Alkylenkette bilden, sowie deren landwirtschaftlich brauchbaren Salze.

Aus der Literatur EP-A 159 117, EP-A 206 523, Indian. J. Chem. 13, 655 (1975) sowie DE-A 33 32 633 sind Pyrazol-3-carbonsäureamide prinzipiell bekannt. Lediglich Indian J. Chem. (loc. cit.) offenbart explizit Pyrazol-3-carbonsäureamide, wobei jedoch ausschließlich Anilide genannt sind.

Der vorliegenden Erfindung lagen neue herbizid wirksame Substanzen als Aufgabe zugrunde.

Demgemäß wurden die eingangs definierten Pyrazol-3-carbonsäureamide I und Verfahren zu ihrer Herstellung gefunden.

Des weiteren wurden gefunden, daß sich die Pyrazol-3-carbonsäureamide I zur Bekämpfung unerwünschten Pflanzenwuchses eignen.

Die erfindungsgemäßen Pyrazol-3-carbonsäureamide I sind auf verschiedenen Wegen herstellbar. Man erhält sie beispieisweise nach dem folgenden Verfahren.

1. Verfahren zur Herstellung der Verbindungen I, in denen $R^5$ eine Gruppe $CO_2R'$ und R' $C_1$-$C_4$-Alkyl bedeutet

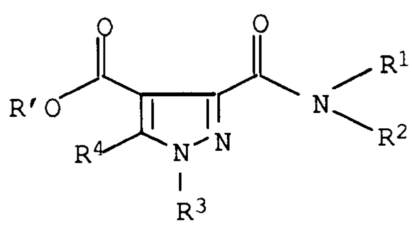

I
($R' = C_1-C_4$-Alkyl)

Man erhält diese Pyrazol-3-carbonsäureamide I dadurch, daß man einen Pyrazol-3,4-dicarbonsäuredialkylester der Formel II in an sich bekannter Weise in einem inerten organischen Lösungsmittel mit

einem Amin der Formel III umsetzt.

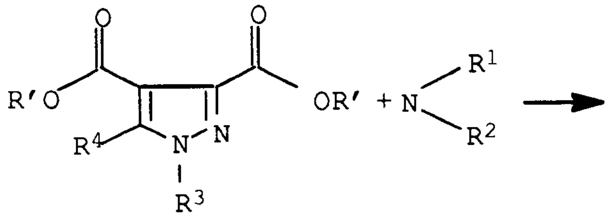

II III I ($R' = C_1-C_4$-Alkyl)

R' in Formel II und I bedeutet eine $C_1$-$C_4$-Alkylgruppe wie Methyl, Ethyl, Propyl, 1-Methylethyl, Butyl, 1-Methylpropyl, 2-Methylpropyl und 1,1-Dimethylethyl, vorzugsweise Methyl, Ethyl und 1-Methylethyl.

Die Umsetzung wird in der Regel bei Temperaturen von 0 bis 100 °C, vorzugsweise 50 bis 80 °C in einem inerten organischen Lösungsmittel durchgeführt.

Als Lösungsmittel eignen sich Halogenkohlenwasserstoffe wie Chlorbenzol und 1,2-Dichlorbenzol, Ether wie Methyl-tert.-butylether, 1,2-Dimethoxyethan, Diethylenglykol-dimethylether, Tetrahydrofuran und Dioxan; Alkohole wie Methanol, Ethanol, Propanol oder Ethylenglykol, dipolare aprotische Lösungsmittel wie Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolin-2-on oder Aromaten wie Benzol,Toluol und Xylol. Besonders bevorzugt arbeitet man in dem der Esterkomponente entsprechenden Alkohol.

Die Konzentration der Edukte im Lösungsmittel beträgt im allgemeinen 0,1 bis 5,0 mol/l, bevorzugt 0,2 bis 2,0 mol/l.

Das molare Verhältnis von II zu III beträgt im allgemeinen 1:2,5 bis 1:1,vorzugsweise 1:1,5 bis 1:1.

Besonders bevorzugt arbeitet man beim Einsatz von Diester II und Amin III im Verhältnis von 1:1 in Abwesenheit von Lösungsmitteln bei Temperaturen von 50 bis 80 °C.

Die als Ausgangsmaterialien für dieses Verfahren benötigten Pyrazoldicarbonsäureester II sind literaturbekannt oder können nach literaturbekannten Verfahren (vgl.: J. Heterocyclic Chem. 25, 1293 (1988); J. Am. Chem. Soc. 73, 3684 (1951); J. Org. Chem. 31, 2491 (1966); J. Heterocyclic Chem. 22, 565 (1985); Chem. Ber. 101, 536 (1968); Chem. Ber. 101, 1059 (1968); Chem. Ber. 107, 3036 (1974) hergestellt werden.

Die benötigten Amine V sind entweder kommerziell erhältlich oder können nach den üblichen Verfahren hergestellt werden.

Ein besonders bevorzugtes Verfahren zur Herstellung der Pyrazol-3-carbon-säureamide der Formel I besteht darin, daß man einen Pyrazol-3,4-dicarbonsäureester der Formel IIa mit wäßriger Base in eine Monocarbonsäure IIb überführt. Die Verbindungen IIb können über die Säurechlorid-Zwischenstufen mit Aminen der Formel III zu den gewünschten Amiden umgesetzt werden.

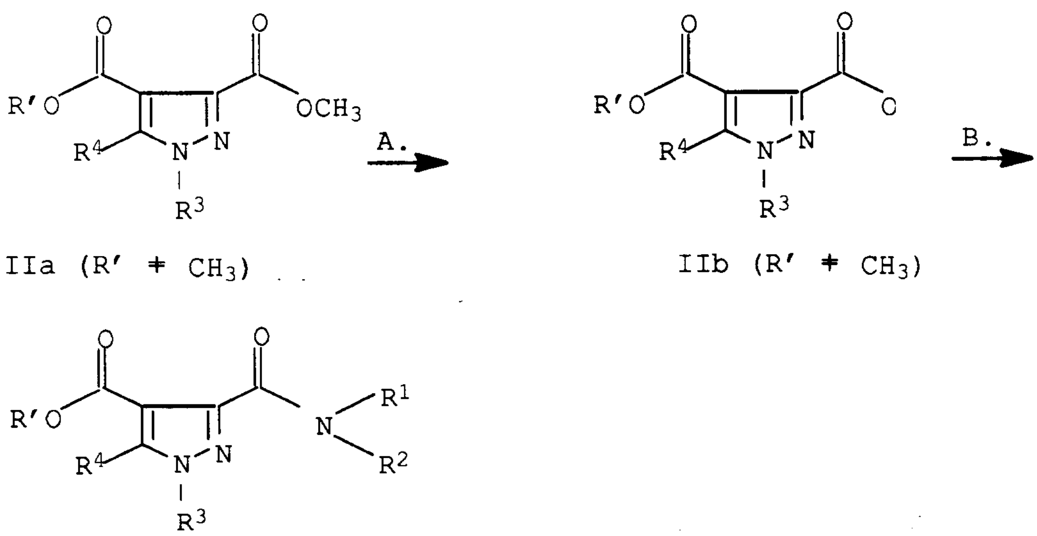


IIa ($R' \neq CH_3$) IIb ($R' \neq CH_3$)

I ($R^5 = CO_2R'$; $R' \neq CH_3$)

R' steht für $C_1$-$C_4$-Alkyl, wie Methyl, Ethyl, Propyl, iso-Propyl, n-Butyl, sek.-Butyl, vorzugsweise Ethyl und iso-Propyl.

Die Reaktion wird so durchgeführt, daß man einen Pyrazol-dicarbonsäureester IIa in einem inerten Lösungsmittel vorlegt und bei -30 °C bis 120 °C; vorzugsweise bei -10 °C bis 40 °C, mit einer wäßrigen Base umsetzt. Die Pyrazol-mono-carbonsäuren der Formel IIb werden dann bei -30 °C bis 100 °C; vorzugsweise bei -10 °C bis 10 °C, durch Zugabe von Mineralsäure freigesetzt.

Als Lösungsmittel kommen Alkohole wie Methanol, Ethanol, Propanol oder Ethylenglykol in Betracht; besonders bevorzugt arbeitet man in dem gleichen Alkohol, der der Esterkomponente in II entspricht. Die Konzentration des Edukts II beträgt im allgemeinen 0,1 bis 0,5 mol/l, bevorzugt 0,2 bis 2,0 mol/l.

Als wäßrige Base setzt man wäßrige Lösungen von Alkali- oder Erdalkalihydroxiden, wie LiOH, NaOH, KOH, $Ca(OH)_2$ oder $Ba(OH)_2$, bevorzugt NaOH oder KOH ein. Die Hyroxide werden dabei in Form einer 5 bis 20 %igen wäßrigen Lösung eingesetzt.

Die molaren Verhältnisse, in denen die Diester IIa und Hydroxide eingesetzt werden, betragen 1:0,95 bis 1:1 für Alkalimetallhydroxide und 1:0,48 bis 1:0,55 für Erdalkalimetallhydroxide.

Die Reaktion ist im allgemeinen nach 14 Stunden beendet; die Mono-carbonsäuren IIb werden dann durch Zugabe einer starken Mineralsäure wie Salzsäure oder Schwefelsäure freigesetzt und auf übliche Art und Weise z.B. durch Absaugen oder Extraktion mit einem organischen Lösungsmittel isoliert.

Zur Überführung der Carbonsäuren IIb in die Säurechloride bringt man die Säuren IIb mit einem Halogenierungsmittel, bevorzugt einem anorganischen Säurehalogenid, gegebenenfalls in einem inerten Lösungsmittel, unter Zugabe katalytischer Mengen von N,N-Dimethylformamid oder 4-N,N-Dimethylaminopyridin, bei 0 °C bis zum Siedepunkt des verwendeten Lösungsmittels(-gemisches) oder Halogenierungsmittels zur Reaktion.

Als inerte Lösungsmittel kommen Halogenkohlenwasserstoffe wie Tetrachlorethan, Methylenchlorid, Chloroform, Dichlorethan, Chlorbenzol und 1,2-Dichlorbenzol, oder Aromaten wie Benzol, Toluol oder Xylol, bevorzugt Benzol oder Toluol in Betracht.

Als Halogenierungsmittel verwendet man zweckmäßigerweise anorganische Säurehalogenide wie Thionylchlorid, Phosphortrichlorid oder Phosphorpentachlorid. Carbonsäuren IIb und Halogenierungsmittel werden im Verhältnis 1:1 bis 1:10, bevorzugt 1:1 bis 1:5 eingesetzt. Die Konzentration des Katalysators (N,N-Dimethylformamid oder 4-N,N-Dimethylaminopyridin) beträgt 1 bis 20 mol-%, vorzugsweise 1 bis 5 mol-%, bezogen auf eingesetze Carbonsäure IIb.

Arbeitet man in einem Lösungsmittel, beträgt die Konzentration des Eduktes IIb 0,1 bis 5,0 mol/l, bevorzugt 0,2 bis 2,0 mol/l.

Besonders bevorzugt arbeitet man mit fünf Äquivalenten Thionylchlorid in Gegenwart von 2 mol-% N,N-Dimethylformamid bei Rückflußtemperatur des Reaktionsgemisches.

Die Reaktion ist im allgemeinen nach 5 Stunden beendet; das Säurechlorid IV kann auf übiche Art und Weise, z.B. durch Abdestillieren des Überschusses an anorganischem Säurehalogenid und des verwendeten Lösungsmittels und nachfolgende Destillation des verbliebenen Säurehalogenids, gegebenenfalls unter vermindertem Druck, isoliert werden.

Die Amide I erhält man in an sich bekannter Weise, indem man ein Pyrazol-3-carbonsäurechlorid der Formel IV mit einem Amin III umsetzt. Dabei geht man zweckmäßigerweise so vor, daß man das Carbonsäurehalogenid in einem inerten Lösungsmittel löst und mit einem Amin III, ebenfalls gelöst in einem inerten Lösungsmittel, umsetzt. Dabei setzt man Säurechlorid und Amin III zweckmäßigerweise im Verhältnis 1:2 bis 1:5 ein; man kann aber auch in Gegenwart eines Säureakzeptors arbeiten, dann setzt man Säurechlorid und Amin III zweckmäßigerweise im Verhältnis 1:1 bis 1:1,5 und Säurechlorid und Säureakzeptor im Verhältnis 1:1,5 bis 1:5 ein.

Als Lösungsmittel verwendet man für diese Umsetzungen vorzugsweise Halogenkohlenwasserstoffe wie Tetrachlorethan, Methylenchlorid, Chloroform, Dichlorethan, Chlorbenzol und 1,2-Dichlorbenzol, Ether, z.B. Diethylether, Methyl-tert.-butylether, 1,2-Dimethoxyethan, Diethylenglykol-dimethylether, Tetrahydrofuran und Dioxan; dipolare aprotische Lösungsmitel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolin-2-on; Aromaten, z.B. Benzol, Toluol und Xylol, oder Ketone wie Aceton und Methylethylketon. Die Konzentration der Edukte im Lösungsmittel beträgt im allgemeinen 0,1 bis 5,0 mol/l, bevorzugt 0,2 bis 2,0 mol/l.

Die Umsetzung kann bei Tempraturen von -30 °C bis zur Rückflußtemperatur des verwendeten Lösungsmittels(-gemisches) durchgeführt werden.

Als Säureakzeptoren setzt man vorzugsweise aromatische Stickstoffbasen, wie Pyridin, 4-Dimethylaminopyridin und Chinolin; tertiäre aliphatische Amine, wie Triethylamin, N-Ethyl-diisopropylamin und N-Methylmorpholin; bi- und tricyclische Amine, wie Diazabicyclooctan (DABCO) und Diazabicycloundecan

(DBU); oder auch Carbonate bzw. Hydrogencarbonate von Alkali- oder Erdalkalimetallen, wie z.B. Natriumcarbonat, Kaliumcarbonat, Natriumhydrogencarbonat und Kaliumhydrogencarbonat ein. Mitunter ist es auch nützlich Kombinationen der oben angeführten Basen zu verwenden.

Besonders bevorzugt arbeitet man in Halogenkohlenwasserstoffen wie Tetrachlorethan, Methylenchlorid, Chloroform und Dichlorethan in Gegenwart von drei Äquivalenten Amin III bei 0 bis 30 ° C.

Die Reaktion ist im allgemeinen nach 14 Stunden beendet; das Gemisch kann wie üblich aufgearbeitet werden, beispielsweise durch Hydrolyse mit Wasser und Extraktion es Amids I mit einem organischen Lösungsmittel. Zur Reinigung kann das Produkt der Formel I wie üblich umkristallisiert oder chromatographiert werden.

2. Verfahren zur Herstellung der Verbindungen I, in denen R5 eine Gruppe $CO_2H$ bedeutet

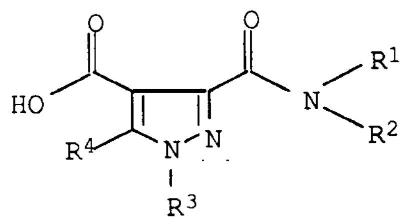

I

($R^5$ = $CO_2H$)

Man erhält diese Verbindungen I dadurch, daß man ein Pyrazol-3-carbon-säureamid I, in dem R5 eine Gruppe $CO_2R'$ und R' $C_1$-$C_4$-Alkyl bedeutet, in an sich bekannter Weise in Gegenwart einer wäßrigen Base hydrolysiert.

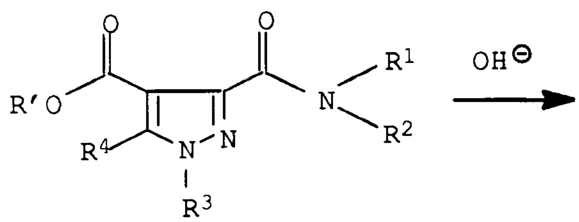


I ($R^5$ = $CO_2H$)

I ($R^5$ = $CO_2R'$)

R' in Formel I hat die vorstehend bei Verfahren 1 angegebene Bedeutung, vorzugsweise jedoch Methyl.

Die Reaktion wird so durchgeführt, daß man einen Pyrazolcarbonsäureester I ($R^5$ = $CO_2R'$) in einem inerten Lösungsmittel vorlegt und bei -30 bis 120 ° C, vorzugsweise bei 10 bis 80 ° C, mit einer wäßrigen Base umsetzt. Die Pyrazolcarbonsäuren der Formel I ($R^5$ = $CO_2H$) werden dann bei -30 bis 100 ° C, vorzugsweise bei -10 bis 10 ° C, durch Zugabe von Mineralsäuren freigesetzt.

Als Lösungsmittel kommen Alkohole wie Methanol, Ethanol, Propanol oder Ethylenglykol in Betracht.

Die Konzentration des Edukts I beträgt im allgemeinen 0,1 bis 5,0 mol/l, bevorzugt 0,2 bis 2,0 mol/l.

Als wäßrige Base setzt man wäßrige Lösungen von Alkali- oder Erdalkalihydroxiden, wie LiOH, NaOH, KOH, $Ca(OH)_2$ oder $Ba(OH)_2$, bevorzugt NaOH oder KOH ein. Die Hydroxide werden dabei in Form einer 5 bis 20 %igen wäßrigen Lösung verwendet.

Die molaren Verhältnisse in denen Ester I und Hydroxide eingesetzt werden, betragen 1:0,95 bis 1:1,1 für Alkalimetallhydroxide und 1:0,48 bis 1:0,55 für Erdalkalimetallhydroxide.

3. Verfahren zur Herstellung der Verbindungen I, in denen $R^5$ eine Gruppe $COYR^6$ bedeutet

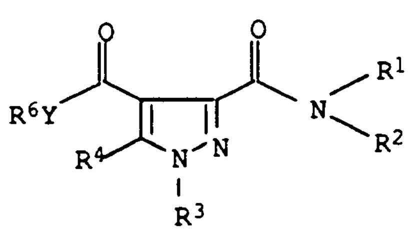

I

($R^5$ = $COYR^6$)

Man erhält diese Verbindungen beispielsweise, wenn man ein Pyrazol-3-carbonsäureamid I, in dem $R^4$

eine Gruppe $CO_2H$ bedeutet in an sich bekannter Weise in oder eine aktivierte Form der Carbonsäure überführt und diese Derivate anschließend mit einer Verbindung V verestert.

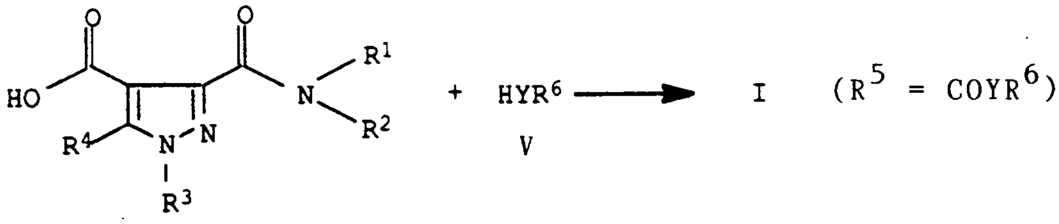

I ($R^5$ = $CO_2H$) V I ($R^5$ = $COYR^6$)

Diese Umsetzung wird üblicherweise bei Temperaturen von -20 bis 60 °C, vorzugsweise 0 bis 40 °C durchgeführt.

Als Lösungsmittel verwendet man zweckmäßigerweise Halogenkohlenwasserstoffe wie Tetrachlormethan, Methylenchlorid, Chloroform, Dichlorethan, Chlorbenzol und 1,2-Dichlorbenzol, Ether, z.B. Methyl-tert.-butylether, 1,2-Dimethoxyethan, Diethylenglykol-dimethylether, Tetrahydrofuran und Dioxan; dipolare aprotische Lösungsmittel, z.B. Acetonitril, Dimethylformamid, Dimethylacetamid, Dimethylsulfoxid, N-Methylpyrrolidon, 1,3-Di-methyltetrahydro-2(1H)-pyrimidinon und 1,3-Dimethylimidazolin-2-on oder Aromaten, z.B. Benzol, Toluol und Xylol. Die Konzentration der Edukte im Lösungsmittel beträgt im allgemeinen 0,1 bis 5,0 mol/l, bevorzugt 0,2 bis 2,0 mol/l.

In der Regel werden 1 bis 1,5 mol-äq., vorzugsweise 1 bis 1,15 mol-äq. der Verbindung IV, bezogen auf die Carbonsäure I ($R^5$ = $CO_2H$), eingesetzt.

Als wasserentziehendes Mittel eignen sich Diimide wie Dicyclohexylcarbodiimid oder Anhydride wie Propanphosphonsäureanhydrid. Auch in Anwesenheit von 1-Methyl-2-halogenpyridiniumiodiden als wasserentziehendes Mittel (vgl. Chem. Lett., 1045 (1975); ibid., 13 (1976); ibid., 49 (1976) gelingt die Umsetzung.

Besonders bevorzugt arbeitet man in einem inerten Lösungsmittel wie Tetrahydrofuran, Dichlormethan oder Toluol in Gegenwart von Dicyclohexylcarbodiimid als wasserentziehendem Mittel beim Einsatz von Carbonsäure I, Verbindung IV und wasserentziehendem Mittel in stöchiometrischen Mengen bei 20 bis 40 °C.

Die Umsetzung ist im allgemeinen nach 14 Stunden beendet; die Pyrazol-3-carbonsäureamide werden in an sich bekannter Weise (z.B. durch Verdünnen des Reaktionsgemisches mit Wasser und Extraktion des Produktes mit einem organischen Lösungsmittel) isoliert und mit üblichen Standardmethoden wie Umkristallisation oder Chromatographie gereinigt.

Im Hinblick auf die bestimmungsgemäße Verwendung der Verbindungen I kommen als Substituenten bevorzugt folgende Reste in Betracht:

$R^1$ und $R^4$

Wasserstoff;

$R^2$

$C_3$-$C_8$-Cycloalkyl wie Cyclopropyl, Cyclobutyl, Cyclopentyl, Cyclohexyl, Cycloheptyl und Cyclooctyl, insbesondere Cyclopropyl, Cyclobutyl, Cyclopentyl und Cyclohexyl; $C_1$-$C_4$-Alkyl wie Methyl, Ethyl, Propyl, 1-Methylethyl, Butyl, 1-Methylpropyl, 2-Methylpropyl und 1,1-Dimethylethyl, insbesondere Methyl und Ethyl;

$R^3$

Wasserstoff;

$C_1$-$C_4$-Alkyl wie voranstehend genannt;

Phenyl, welches ein bis drei der folgenden Reste tragen kann:

Halogen wie insbesondere Fluor und Chlor; Alkyl wie insbesondere Methyl, Ethyl und 1-Methylethyl; Halogenalkyl wie insbesondere Trifluorethyl; Alkoxy wie insbesondere Methoxy, Ethoxy und 1-Methylethoxy; Halogenalkoxy wie insbesondere Difluormethoxy und Trifluormethoxy; Alkylthio wie insbesondere Methylthio und Ethylthio;

$R^5$

eine Grupe $COYR^6$;

Y

Sauerstoff

$R^6$

Wasserstoff oder eine Gruppe $N=CR^7R^8$, wobei

$R^7$

Wasserstoff oder Alkyl wie voranstehend genannt, insbesondere Methyl, Ethyl und 1-Methylethyl und

$R^8$

Cycloalkyl wie insbesondere Cyclopropyl, Phenyl, Furyl oder eine der bei $R^7$ genannten Gruppen bedeutet oder

$R^7$, $R^8$

gemeinsam eine Alkylenkette wie Butylen, Pentylen, Hexylen und Heptylen, insbesondere Butylen und Pentylen bilden,

sowie deren landwirtschaftlich brauchbaren Salze.

Besonders bevorzugt sind Verbindungen I, in denen die Substituenten folgende Bedeutung haben:

$R^1$

Wasserstoff;

$R^2$

Alkyl wie insbesondere Methyl, Ethyl, Propyl, 1-Methylethyl und 1,1-Dimethylethyl oder Cycloalkyl wie insbesondere Cyclopropyl, Cyclopentyl und Cyclohexyl;

$R^3$

Wasserstoff;

Alkyl wie insbesondere Methyl, Ethyl, Propyl und 1-Methylethyl, oder

Phenyl, welches ein bis drei der folgenden Reste tragen kann: Halogen wie insbesondere Fluor und Chlor, Alkyl wie insbesondere Methyl, Ethyl und 1-Methylethyl, Halogenalkyl wie insbesondere Trifluormethyl, Halogenalkoxy wie insbesondere Trifluormethoxy, Alkylthio wie insbesondere Methylthio und/oder Halogenalkylthio wie insbesondere Trifluormethylthio;

$R^4$

Wasserstoff;

$R^5$

eine Gruppe $COYR^6$;

Y

Sauerstoff;

$R^6$

Wasserstoff oder eine Gruppe $-N=R^7R^8$ und

$R^7$

Wasserstoff oder Alkyl wie bei $R^3$ genannt;

$R^8$

Cycloalkyl wie insbesondere bei $R^2$ genannt oder eine der bei $R^7$ genannten Gruppen, oder

$R^7$, $R^8$

gemeinsam eine Alkylenkette wie insbesondere Butylen und Pentylen.

Beispiele für insbesonders bevorzugte Pyrazol-3-carbonsäureamide der allgemeinen Formel I sind in der folgenden Tabelle aufgeführt.

Tabelle

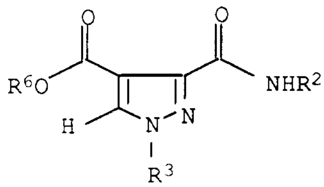

| $R^3$ | $R^2$ | $R^6$ |
|---|---|---|
| Methyl | tert.-Butyl | H |
| Ethyl | tert.-Butyl | H |
| n-Propyl | tert.-Butyl | H |
| iso-Propyl | tert.-Butyl | H |
| n-Butyl | tert.-Butyl | H |
| iso-Butyl | tert.-Butyl | H |
| sek.-Butyl | tert.-Butyl | H |
| tert.-Butyl | tert.-Butyl | H |
| Phenyl | tert.-Butyl | H |
| 4-F-Phenyl | tert.-Butyl | H |
| 3-Phenyl | tert.-Butyl | H |
| 2-F-Phenyl | tert.-Butyl | H |
| 4-Cl-Phenyl | tert.-Butyl | H |
| 2-Cl-Phenyl | tert.-Butyl | H |
| 2,4-(Cl,Cl)-Phenyl | tert.-Butyl | H |
| 2,4-(F,Cl)-Phenyl | tert.-Butyl | H |
| 2,4,6-(Cl,Cl,Cl)-Phenyl | tert.-Butyl | H |
| 4-$CF_3$-Phenyl | tert.-Butyl | H |
| 3-$CF_3$-Phenyl | tert.-Butyl | H |
| 2,4,6-(Cl,$CF_3$,Cl)-Phenyl | tert.-Butyl | H |
| 2,4,6-(F,$CF_3$,F)-Phenyl | tert.-Butyl | H |
| 4-$CCl_3$-Phenyl | tert.-Butyl | H |
| 4-$CH_3$-Phenyl | tert.-Butyl | H |
| 2,4,6-Trimethylphenyl | tert.-Butyl | H |
| 4-t-Butylphenyl | tert.-Butyl | H |
| 2-iso-Propylphenyl | tert.-Butyl | H |
| 4-iso-Propylphenyl | tert.-Butyl | H |
| 4-Propylphenyl | tert.-Butyl | H |
| 2-$CF_3$-Phenyl | tert.-Butyl | H |
| 2-$OCH_3$-Phenyl | tert.-Butyl | H |
| 4-$OCH_3$-Phenyl | tert.-Butyl | H |

| $R^3$ | $R^2$ | $R^6$ |
|---|---|---|
| 2,4-($OCH_3$,$OCH_3$)-Phenyl | tert.-Butyl | H |
| 3-$OCF_3$-Phenyl | tert.-Butyl | H |
| 3-$OCClF_2$-Phenyl | tert.-Butyl | H |
| 4-$OCHF_2$-Phenyl | tert.-Butyl | H |
| 2-$SCH_3$-Phenyl | tert.-Butyl | H |
| 4-$SCH_3$-Phenyl | tert.-Butyl | H |
| Methyl | cyclo-Propyl | H |
| Ethyl | cyclo-Propyl | H |
| n-Propyl | cyclo-Propyl | H |
| iso-Propyl | cyclo-Propyl | H |
| n-Butyl | cyclo-Propyl | H |
| iso-Butyl | cyclo-Propyl | H |
| sek.-Butyl | cyclo-Propyl | H |
| tert.-Butyl | cyclo-Propyl | H |
| Phenyl | cyclo-Propyl | H |
| 4-F-Phenyl | cyclo-Propyl | H |
| 3-F-Phenyl | cyclo-Propyl | H |
| 2-F-Phenyl | cyclo-Propyl | H |
| 4-Cl-Phenyl | cyclo-Propyl | H |
| 2-Cl-Phenyl | cyclo-Propyl | H |
| 2,4-(Cl,Cl)-Phenyl | cyclo-Propyl | H |
| 2,4-(F,Cl)-Phenyl | cyclo-Propyl | H |
| 2,4,6-(Cl,Cl,Cl)-Phenyl | cyclo-Propyl | H |
| 4-$CF_3$-Phenyl | cyclo-Propyl | H |
| 3-$CF_3$-Phenyl | cyclo-Propyl | H |
| 2,4,6-(Cl,$CF_3$,Cl)-Phenyl | cyclo-Propyl | H |
| 2,4,6-(F,$CF_3$,F)-Phenyl | cyclo-Propyl | H |
| 4-$CCl_3$-Phenyl | cyclo-Propyl | H |
| 4-$CH_3$-Phenyl | cyclo-Propyl | H |
| 2,4,6-Trimethylphenyl | cyclo-Propyl | H |
| 4-t-Butylphenyl | cyclo-Propyl | H |
| 2-iso-Propylphenyl | cyclo-Propyl | H |
| 4-iso-Propylphenyl | cyclo-Propyl | H |
| 4-Propylphenyl | cyclo-Propyl | H |
| 2-$CF_3$-Phenyl | cyclo-Propyl | H |
| 2-$OCH_3$-Phenyl | cyclo-Propyl | H |
| 4-$OCH_3$-Phenyl | cyclo-Propyl | H |
| 2,4-($OCH_3$,$OCH_3$)-Phenyl | cyclo-Propyl | H |
| 3-$OCF_3$-Phenyl | cyclo-Propyl | H |

| $R^3$ | $R^2$ | $R^6$ |
|---|---|---|
| 3-$OCClF_2$-Phenyl | cyclo-Propyl | H |
| 4-$OCHF_2$-Phenyl | cyclo-Propyl | H |
| 2-$SCH_3$-Phenyl | cyclo-Propyl | H |
| 4-$SCH_3$-Phenyl | cyclo-Propyl | H |
| H | tert.-Butyl | $-N=C(CH_3)_2$ |
| Methyl | tert.-Butyl | $-N=C(CH_3)_2$ |
| Ethyl | tert.-Butyl | $-N=C(CH_3)_2$ |
| n-Propyl | tert.-Butyl | $-N=C(CH_3)_2$ |
| iso-Propyl | tert.-Butyl | $-N=C(CH_3)_2$ |
| n-Butyl | tert.-Butyl | $-N=C(CH_3)_2$ |
| iso-Butyl | tert.-Butyl | $-N=C(CH_3)_2$ |
| sek.-Butyl | tert.-Butyl | $-N=C(CH_3)_2$ |
| tert.-Butyl | tert.-Butyl | $-N=C(CH_3)_2$ |
| Phenyl | tert.-Butyl | $-N=C(CH_3)_2$ |
| 4-F-Phenyl | tert.-Butyl | $-N=C(CH_3)_2$ |
| 3-F-Phenyl | tert.-Butyl | $-N=C(CH_3)_2$ |
| 2-F-Phenyl | tert.-Butyl | $-N=C(CH_3)_2$ |
| 4-Cl-Phenyl | tert.-Butyl | $-N=C(CH_3)_2$ |
| 2-Cl-Phenyl | tert.-Butyl | $-N=C(CH_3)_2$ |
| 2,4-(Cl,Cl)-Phenyl | tert.-Butyl | $-N=C(CH_3)_2$ |
| 2,4-(F,Cl)-Phenyl | tert.-Butyl | $-N=C(CH_3)_2$ |
| 2,4,6-(Cl,Cl,Cl)-Phenyl | tert.-Butyl | $-N=C(CH_3)_2$ |
| 4-$CF_3$-Phenyl | tert.-Butyl | $-N=C(CH_3)_2$ |
| 3-$CF_3$-Phenyl | tert.-Butyl | $-N=C(CH_3)_2$ |
| 2,4,6-(Cl,$CF_3$,Cl)-Phenyl | tert.-Butyl | $-N=C(CH_3)_2$ |
| 2,4,6-(F,$CF_3$,F)-Phenyl | tert.-Butyl | $-N=C(CH_3)_2$ |
| 4-$CCl_3$-Phenyl | tert.-Butyl | $-N=C(CH_3)_2$ |
| 4-$CH_3$-Phenyl | tert.-Butyl | $-N=C(CH_3)_2$ |
| 2,4,6-Trimethylphenyl | tert.-Butyl | $-N=C(CH_3)_2$ |
| 4-t-Butylphenyl | tert.-Butyl | $-N=C(CH_3)_2$ |
| 2-iso-Propylphenyl | tert.-Butyl | $-N=C(CH_3)_2$ |
| 4-iso-Propylphenyl | tert.-Butyl | $-N=C(CH_3)_2$ |
| 4-Propylphenyl | tert.-Butyl | $-N=C(CH_3)_2$ |
| 2-$CF_3$-Phenyl | tert.-Butyl | $-N=C(CH_3)_2$ |
| 2-$OCH_3$-Phenyl | tert.-Butyl | $-N=C(CH_3)_2$ |
| 4-$OCH_3$-Phenyl | tert.-Butyl | $-N=C(CH_3)_2$ |
| 2,4-($OCH_3$,$OCH_3$)-Phenyl | tert.-Butyl | $-N=C(CH_3)_2$ |
| 3-$OCF_3$-Phenyl | tert.-Butyl | $-N=C(CH_3)_2$ |
| 3-$OCClF_2$-Phenyl | tert.-Butyl | $-N=C(CH_3)_2$ |

| $R^3$ | $R^2$ | $R^6$ |
|---|---|---|
| 4-$OCHF_2$-Phenyl | tert.-Butyl | $-N=C(CH_3)_2$ |
| 2-$SCH_3$-Phenyl | tert.-Butyl | $-N=C(CH_3)_2$ |
| 4-$SCH_3$-Phenyl | tert.-Butyl | $-N=C(CH_3)_2$ |
| H | cyclo-Propyl | $-N=C(CH_3)_2$ |
| Methyl | cyclo-Propyl | $-N=C(CH_3)_2$ |
| Ethyl | cyclo-Propyl | $-N=C(CH_3)_2$ |
| n-Propyl | cyclo-Propyl | $-N=C(CH_3)_2$ |
| iso-Propyl | cyclo-Propyl | $-N=C(CH_3)_2$ |
| n-Butyl | cyclo-Propyl | $-N=C(CH_3)_2$ |
| iso-Butyl | cyclo-Propyl | $-N=C(CH_3)_2$ |
| sek.-Butyl | cyclo-Propyl | $-N=C(CH_3)_2$ |
| tert.-Butyl | cyclo-Propyl | $-N=C(CH_3)_2$ |
| Phenyl | cyclo-Propyl | $-N=C(CH_3)_2$ |
| 4-F-Phenyl | cyclo-Propyl | $-N=C(CH_3)_2$ |
| 3-F-Phenyl | cyclo-Propyl | $-N=C(CH_3)_2$ |
| 2-F-Phenyl | cyclo-Propyl | $-N=C(CH_3)_2$ |
| 4-Cl-Phenyl | cyclo-Propyl | $-N=C(CH_3)_2$ |
| 2-Cl-Phenyl | cyclo-Propyl | $-N=C(CH_3)_2$ |
| 2,4-(Cl,Cl)-Phenyl | cyclo-Propyl | $-N=C(CH_3)_2$ |
| 2,4-(F,Cl)-Phenyl | cyclo-Propyl | $-N=C(CH_3)_2$ |
| 2,4,6-(Cl,Cl,Cl)-Phenyl | cyclo-Propyl | $-N=C(CH_3)_2$ |
| 4-$CF_3$-Phenyl | cyclo-Propyl | $-N=C(CH_3)_2$ |
| 3-$CF_3$-Phenyl | cyclo-Propyl | $-N=C(CH_3)_2$ |
| 2,4,6-(Cl,$CF_3$,Cl)-Phenyl | cyclo-Propyl | $-N=C(CH_3)_2$ |
| 2,4,6-(F,$CF_3$,F)-Phenyl | cyclo-Propyl | $-N=C(CH_3)_2$ |
| 4-$CCl_3$-Phenyl | cyclo-Propyl | $-N=C(CH_3)_2$ |
| 4-$CH_3$-Phenyl | cyclo-Propyl | $-N=C(CH_3)_2$ |
| 2,4,6-Trimethylphenyl | cyclo-Propyl | $-N=C(CH_3)_2$ |
| 4-t-Butylphenyl | cyclo-Propyl | $-N=C(CH_3)_2$ |
| 2-iso-Propylphenyl | cyclo-Propyl | $-N=C(CH_3)_2$ |
| 4-iso-Propylphenyl | cyclo-Propyl | $-N=C(CH_3)_2$ |
| 4-Propylphenyl | cyclo-Propyl | $-N=C(CH_3)_2$ |
| 2-$CF_3$-Phenyl | cyclo-Propyl | $-N=C(CH_3)_2$ |
| 2-$OCH_3$-Phenyl | cyclo-Propyl | $-N=C(CH_3)_2$ |
| 4-$OCH_3$-Phenyl | cyclo-Propyl | $-N=C(CH_3)_2$ |
| 2,4-($OCH_3$,$OCH_3$)-Phenyl | cyclo-Propyl | $-N=C(CH_3)_2$ |
| 3-$OCF_3$-Phenyl | cyclo-Propyl | $-N=C(CH_3)_2$ |
| 3-$OCClF_2$-Phenyl | cyclo-Propyl | $-N=C(CH_3)_2$ |
| 4-$OCHF_2$-Phenyl | cyclo-Propyl | $-N=C(CH_3)_2$ |

| $R^3$ | $R^2$ | $R^6$ |
|---|---|---|
| 2-$SCH_3$-Phenyl | cyclo-Propyl | $-N=C(CH_3)_2$ |
| 4-$SCH_3$-Phenyl | cyclo-Propyl | $-N=C(CH_3)_2$ |
| H | cyclo-Propyl | H |
| H | Methyl | H |
| H | Ethyl | H |
| H | n-Propyl | H |
| H | iso-Propyl | H |
| H | n-Butyl | H |
| H | iso-Butyl | H |
| Methyl | iso-Butyl | H |
| Methyl | sek.-Butyl | H |
| Methyl | n-Pentyl | H |
| Methyl | 2-Pentyl | H |
| Methyl | 3-Pentyl | H |
| H | Methyl | $-N=C(CH_3)_2$ |
| H | Ethyl | $-N=C(CH_3)_2$ |
| H | n-Propyl | $-N=C(CH_3)_2$ |
| H | iso-Propyl | $-N=C(CH_3)_2$ |
| H | n-Butyl | $-N=C(CH_3)_2$ |
| Methyl | iso-Butyl | $-N=C(CH_3)_2$ |
| Methyl | sek.-Butyl | $-N=C(CH_3)_2$ |
| H | tert.-Butyl | $N=C(C_2H_5)_2$ |
| H | tert.-Butyl | $N=C(cyclo-C_3H_5)_2$ |
| H | tert.-Butyl | 2-Butanimino |
| H | tert.-Butyl | Cyclohexanimino |
| Methyl | tert.-Butyl | Cyclooctanimino |
| H | cyclo-Propyl | $N=C(C_2H_5)_2$ |
| H | cyclo-Propyl | 2-Butanimino |
| H | cyclo-Propyl | Cyclohexanimino |
| Methyl | cyclo-Propyl | Cyclooctanimino |

Als Salze der Verbindungen der Formel I kommen landwirtschaftlich brauchbare Salze, beispielsweise Alkalimetallsalze, wie das Kalium- oder Natriumsalz, Erdalkalimetallsalze, wie das Calcium-, Magnesium- oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die erfindungsgemäßen herbiziden Verbindungen I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall

möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerteZusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkchole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,01 und 95 Gew.%, vorzugsweise zwischen 0,1 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.014 mit 10 Gewichtsteilen N-Methyl-a-pyrrolidon und erhält eine Lösung, die zur Anwendung in Form kleinster Tropfen geeignet ist.

II. 20 Gewichtsteile der Verbindung Nr. 1.006 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht.

Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile des Wirkstoffs Nr. 1.014 werden in einer-Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feinesVerteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.004 werden mit 3 Gewichtsteilen des Natriumsalzes der Diisobutylnaphthalin-a-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

V. 30 Gewichtsteile des Wirkstoffs Nr. 1.006 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff bei Anwendung als Herbizide betragen je nach Bekämpfungsziel, Jahreszeit, Zielpflanzen und Wachstumsstadium 0,001 bis 5, vorzugsweise 0,01 bis 2 kg/ha aktive Substanz (a.S.).

In Anbetracht der Vielseitigkeit der Applikationsmethoden können die erfindungsgemäßen Verbindungen bzw. sie enthaltende Mittel in einer großen Zahl von Kulturpflanzen zur Beseitigung unerwünschter Pflanzen eingesetzt werden.

Zur Verbreiterung des Wirkungsspektrums und zur Erzielung synergistischer Effekte können die erfindungsgemäßen Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffderivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

Herstellungsbeispiele:

1. 1(H)-4-Ethoxycarbonyl-1-methyl-pyrazol-3-carbonsäure

Eine Lösung von 10,5 g (50 mmol) 1(H)-1-Methyl-pyrazol-3,4-dicarbonsäurediethylester (J. Org. Chem. 31, 2491 (1966)) in 200 ml Ethanol/Wasser (1:1) wurde auf 0 °C abgekühlt und tropfenweise innerhalb von 30 Minuten mit einer Lösung von 1,96 g (0,049 mol) Natronlauge in 50 ml Wasser versetzt. Man ließ das Gemisch 14 Stunden bei Raumtemperatur stehen, entfernte das Lösungsmittelgemisch im Vakuum, nahm den festen Rückstand in 100 ml Wasser auf, säuerte mit 10 %iger Salzsäure an und saugte das ausgefallene Produkt ab. Man erhielt 7,40 g (74 %) Halbester vom Schmelzpunkt 182 °C (aus Wasser) als weißes Pulver.

2. 1(H)-4-Ethoxycarbonyl-1-methyl-pyrazol-3-carbonsäurechlorid

7,20 g (36 mmol) 1(H)-4-Ethoxycarbonyl-1-methyl-pyrazol-3-carbonsäure aus Beispiel 1 wurden portionsweise in 20 ml Thionylchlorid eingetragen und dann noch zwei Stunden zum Rückfluß erhitzt. Anschließend wurde überschüssiges Thionylchlorid im Vakuum entfernt, der Rückstand in 50 ml Diethylether aufgenommen und 30 Minuten mit 2,00 g Aktivkohle gerührt. Man filtrierte ab und engte das Filtrat im Vakuum ein. Als Rückstand behielt man 7,30 g (94 %) Säurechlorid als weißen Feststoff vom Schmelzpunkt: 65 °C zurück.

3. 1(H)-4-Ethoxycarbonyl-1-methyl-pyrazol-3-carbonsäure-iso-propylamid

4,00 g (68 mmol) iso-Propylamin wurden bei 0 °C in 100 ml Dichlormethan gelöst, die erhaltene Lösung auf -10 °C abgekühlt und bei dieser Temperatur innerhalb von 30 Minuten tropfenweise mit einer Lösung von 7,20 g (33 mmol) 1(H)-4-Ethoxycarbonyl-1-methyl-pyrazol-3-carbonsäurechlorid aus Beispiel 2 in 20 ml Dichlormethan versetzt. Nach beendeter Zugabe ließ man das Gemisch auf Raumtemperatur erwärmen, rührte noch zwei Stunden nach und hydrolysierte durch Zugabe von 100 ml 10 %iger Salzsäure. Die organische Phase wurde abgetrennt, mit Natriumsulfat getrocknet und eingeengt. Als Rückstand blieben 7,20 g (91 %) Amid als weißer Feststoff vom Schmelzpunkt: 134 °C (aus Cyclohexan/Essigester = 1:1) zurück.

4. 1(H)-1-Methyl-3-(iso-propylaminocarbonyl)-pyrazol-4-carbonsäure

Eine Mischung aus 7,00 g (29 mmol) 1(H)-4-Ethoxycarbonyl-1-methyl-pyrazol-3-carbonsäure-iso-propylamid aus Beispiel 3 und 1,85 g (33 mmol) Kaliumhydroxid in 60 ml Wasser/Methanol = 1:1 wurde drei Stunden auf 60 °C erhitzt. Danach wurde das Lösungsmittelgemisch im Vakuum entfernt und der feste Rückstand in 20 ml Wasser aufgenommen. Man säuerte mit konzentrierter Salzsäure an, saugte das ausgefallene Produkt ab und erhielt so 6,00 g (98 %) Carbonsäure als weißen Feststoff vom Schmelzpunkt: 180 °C (aus Methanol) (Wirkstoffbeispiel Nr. 1.001).

5. 1(H)-3-(2-Chlorbenzyl-aminocarbonyl)-1-methyl-pyrazol-4-carbonsäure

Eine Mischung von 2,55 g (18 mmol) 2-Chlorbenzylamin und 2,00 g (20 mmol) Triethylamin in 50 ml Dichlormethan wurde auf 0 °C abgekühlt und mit einer Lösung von 4,00 g (18 mmol) 1(H)-4-Ethoxycarbonyl-1-methyl-pyrazol-3-carbonsäurechlorid aus Beispiel 2 in 20 ml Dichlormethan versetzt. Man ließ das Gemisch auf Raumtemperatur erwärmen, rührte noch zwei Stunden nach, hydrolysierte durch Zugabe von 50 ml 10 %iger Salzsäure und trennte die organische Phase ab. Man rotierte ein, nahm den Rückstand mit 50 ml Methanol/Wasser (1:1) auf und gab eine Lösung von 1,12 g (20 mmol) Kaliumhydroxid in 20 ml Wasser zu. Die resultierende Mischung wurde zwei Stunden auf 65 °C erhitzt, dann im Vakuum vom Lösungsmittel befreit und der Rückstand mit 50 ml Wasser aufgenommen. Man säuerte mit konzentrierter Salzsäure an und saugte das ausgefallene Produkt ab. Man erhielt 5,00 g (95 %) Amid vom Schmelzpunkt: 154 °C.

6. 1(H)-1-Methyl-4-(2-propaniminoxycarbonyl)-pyrazol-3-carbonsäure-tert.-butylamid

Eine Suspension von 6,12 g (24 mmol) 1-Methyl-2-chlor-pyridiniumiodid in 20 ml Dichlormethan wurde innerhalb von 30 Minuten tropfenweise mit einer Mischung von 4,50 g (20 mmol) 1(H)-3-tert.-Butylaminocarbonyl-1-methyl-pyrazol-4-carbonsäure, 1,50 g (20 mmol) Acetonoxim und 4,80 g (48 mmol) Triethylamin in 20 ml Dichlormethan versetzt. Nach beendeter Zugabe erhitzte man drei Stunden zum Rückfluß, entfernte das Lösungsmittel im Vakuum und reinigte den Rückstand durch Chromatographie an Kieselgel (Elutionsmittel:Dichlormethan/Essigester = 5:1). Man erhielt so 4,40 g (80 %) Oximester als weißen Feststoff vom Schmelpunkt 111 °C (Wirkstoffbeispiel Nr. 1.004).

Die in der folgenden Tabelle aufgeführten Verbindungen wurden analog zu den voranstehenden Beispielen hergestellt.

Tabelle 1

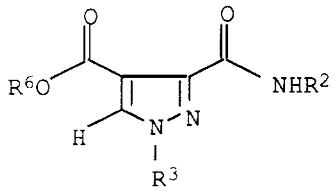

I

| Bsp.-Nr. | $R^3$ | $R^2$ | $R^6$ | Schmelzpunkt [°C] |
|---|---|---|---|---|
| 1.001 | Methyl | iso-Propyl | H | 180 |
| 1.002 | H | iso-Propyl | H | 182 |
| 1.003 | Methyl | tert.-Butyl | H | 149 |
| 1.004 | Methyl | tert.-Butyl | $-N=C(CH_3)_2$ | 111 |
| 1.005 | Methyl | cyclo-Propyl | H | 208 |
| 1.006 | H | tert.-Butyl | H | 200 |
| 1.007 | H | cyclo-Propyl | H | 212 |
| 1.008 | 4-$(CF_3)$-Phenyl | cyclo-Propyl | H | 211 |
| 1.009 | 4-Trifluor-methylphenyl | tert.-Butyl | H | 170 |
| 1.010 | 4-Fluorphenyl | tert.-Butyl | H | 210 |
| 1.011 | 4-Fluorphenyl | cyclo-Propyl | H | 200 |
| 1.012 | 2-(i-Propyl)-phenyl | cyclo-Propyl | H | 68 |
| 1.013 | 2-(i-Propyl)-phenyl | tert.-Butyl | H | 134 |
| 1.014 | 4-(t-Butyl)-phenyl | tert.-Butyl | H | 98 |
| 1.015 | 4-Methoxy-phenyl | tert.-Butyl | H | 133 |
| 1.016 | 2,4,6-Trimethyl-phenyl | tert.-Butyl | H | 164 |
| 1.017 | 2-Methoxy-phenyl | tert.-Butyl | H | 81 |
| 1.018 | 3-Trifluormeth-oxy-phenyl | tert.-Butyl | H | 174 |
| 1.019 | 3-Trifluor-methylphenyl | tert.-Butyl | H | 178 |
| 1.020 | 3,5-Dichlor-2-methyoxy-phenyl | tert.-Butyl | H | 90 |
| 1.021 | 2-Methylthio-phenyl | tert.-Butyl | H | 141 |
| 1.022 | 3-Chlor-4-me-thylthio-phenyl | tert.-Butyl | H | 221 |

| Bsp.-Nr. | $R^3$ | $R^2$ | $R^6$ | Schmelzpunkt [°C] |
|---|---|---|---|---|
| 1.023 | 4-Methylthio-phenyl | tert.-Butyl | H | 143 |
| 1.024 | 2,4-Dimethyl-phenyl | tert.-Butyl | H | 118-120 |
| 1.025 | 2,4-Difluor-phenyl | tert.-Butyl | H | 150-152 |
| 1.026 | 2-Chlor-4-meth-oxy-phenyl | tert.-Butyl | H | 198-200 |
| 1.027 | 4-Chlor-2-me-thyl-phenyl | tert.-Butyl | H | 98-100 |
| 1.028 | 4-Fluor-2-me-thyl-phenyl | tert.-Butyl | H | 144-146 |

Anwendungsbeispiele

Die herbizide Wirkung der Pyrazol-3-carbonsäureamide der Formel I ließ sich durch Gewächshausversuche zeigen:
Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 1,0 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Chenopodium album | Weißer Gänsefuß |
| Sinapis alba | Weißer Senf |
| Solanum nigrum | Schwarzer Nachtschatten |

Mit 1,0 kg/ha a.S. im Nachauflaufverfahren eingesetzt, lassen sich mit den Beispielen 1.004 und 1.006 breitblättrige unerwünschte Pflanzen sehr gut bekämpfen.

**Patentansprüche**

1. Pyrazol-3-carbonsäureamide der allgemeinen Formel I

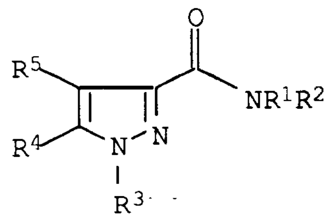

I,

in der die Substituenten folgende Bedeutung haben:

$R^1$ und $R^4$

Wasserstoff;

$R^2$

$C_1$-$C_4$-Alkyl oder $C_3$-$C_8$-Cycloalkyl;

$R^3$

Wasserstoff;

$C_1$-$C_4$-Alkyl;

Phenyl, welches ein bis drei der folgenden Reste tragen kann: Halogen, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

$R^5$

eine Gruppe $COYR^6$;

Y

Sauerstoff;

$R^6$

Wasserstoff, oder eine Gruppe $-N=CR^7R^8$, wobei

$R^7$

Wasserstoff oder $C_1$-$C_4$-Alkyl und

$R^8$

$C_3$-$C_6$-Cycloalkyl oder einen Rest $R^7$ bedeutet oder

$R^7$, $R^8$

gemeinsam eine 4- bis 7-gliedrige Alkylenkette bilden, sowie deren landwirtschaftlich brauchbaren Salze.

2. Verfahren zur Herstellung der Verbindungen I gemäß Anspruch 1, in denen $R^5$ eine Gruppe $CO_2H$ bedeutet, daß man ein Pyrazol-3-carbonsäureamid I, in dem $R^5$ eine Gruppe $CO_2R'$ und R' $C_1$-$C_4$-Alkyl bedeutet

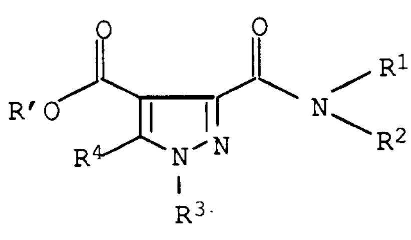

I,
($R^5$ = $CO_2R'$)

in an sich bekannter Weise in Gegenwart einer wäßrigen Base hydrolysiert.

3. Verfahren zur Herstellung der Verbindungen I gemäß Anspruch 1, in denen $R^5$ eine Gruppe $COYR^6$ bedeutet, dadurch gekennzeichnet, daß man ein Pyrazol-3-carbonsäureamid I, in dem $R^5$ eine Gruppe $CO_2H$ bedeutet

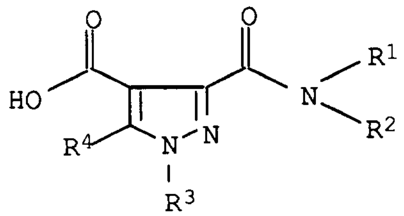

I,

($R^5$ = $CO_2H$)

in an sich bekannter Weise in das Halogenid oder eine andere aktivierte Form der Carbonsäure überführt und diese Derivate anschließend mit einer Verbindung IV

$HYR^6$ V

verestert.

4. Herbizides Mittel, enthaltend mindestens ein Pyrazol-3-carbonsäureamid der allgemeinen Formel I gemäß Anspruch 1 und übliche inerte Zusatzstoffe.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Pyrazol-3-carbonsäureamids I gemäß Anspruch 1 behandelt.

## Claims

1. A pyrazole-3-carboxamide of the general formula I

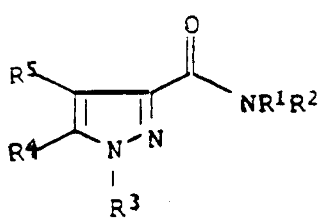

I,

where the substituents have the following meanings:
$R^1$ and $R^4$
hydrogen;
$R^2$
$C_1$-$C_4$-alkyl or $C_3$-$C_8$-cycloalkyl,
$R^3$
hydrogen;
$C_1$-$C_4$-alkyl;
phenyl which may carry from one to three of the following radicals: halogen, $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
$R^5$
a $COYR^6$ group;
Y
oxgen;
$R^6$
hydrogen, or an $-N=CR^7R^8$ group where
$R^7$ is hydrogen or $C_1$-$C_4$-alkyl and
$R^8$ is $C_3$-$C_6$-cycloalkyl or a radical $R^7$, or
$R^7$ and $R^8$ together form a 4- to 7-membered alkylene chain, or an agriculturally useful salt thereof.

2. A process for a preparation of a compound I as claimed in claim 1 in which $R^5$ is $CO_2H$, which comprises hydrolyzing a pyrazole-3-carboxamide I in which $R^5$ is $CO_2R'$ and R' is $C_1$-$C_4$-alkyl

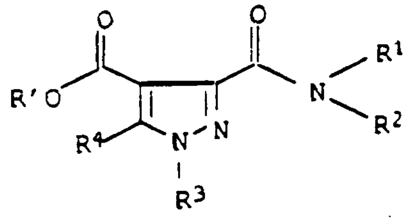

I,

($R^5$ = $CO_2R'$)

in a conventional manner in the presence of an aqueous base.

**3.** A process for the preparation of compound I as claimed in claim 1 in which $R^5$ is $COYR^6$, which comprises converting a pyrazole-3-carboxamide I in which $R^5$ is $CO_2H$

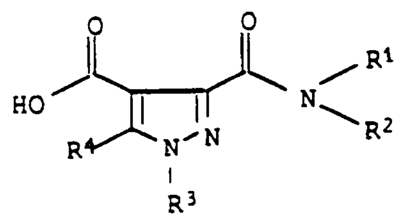

I,

($R^5$ = $CO_2H$)

in a conventional manner into the halide or another activated form of the carboxylic acid and subsequently esterifying the latter using a compound V

$HYR^6$     V

**4.** A herbicide containing at least one pyrazole-3-carboxamide of the general formula I as claimed in claim 1 and conventional inert additives.

**5.** A method of controlling undesired plant growth, wherein the undesired plants and/or their habitat are treated with a herbicidally effective amount of a pyrazole-3-carboxamide I as claimed in claim 1.

## Revendications

**1.** Amide d'acide pyrazol-3-carboxylique de la formule générale I

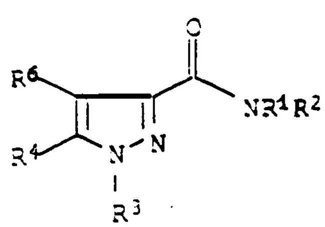

I,

dans laquelle les substituants ont les significations suivantes,

| | |
|---|---|
| $R^1$ et $R^4$ | hydrogène |
| $R^2$ | alkyle en C1-C4 ou cycloalkyle en C3-C8; |
| $R^3$ | hydrogène, alkyle en C1-C4, phényle qui peut porter un à trois des restes suivants : halogène, alkyle en C1-C4, halogénalkyle en C1-C4, alcoxy en C1-C4, halogénalcoxy en C1-C4 et/ou alkylthio en C1-C4; |
| $R^5$ | un groupe $COYR^6$; |
| Y | oxygène; |
| $R^6$ | hydrogène ou un groupe $-N-CR^7R^8$; |
| $R^7$ | représentant hydrogène ou alkyle en C1-C4 et |
| $R^8$ | représentant cycloalkyle en C3-C6 ou un reste $R^7$, ou $R^7$, $R^8$, ensemble formant une |

chaîne alkylène de 4 à 7 chaînons;
ainsi que leurs sels utilisables en agriculture.

2. Procédé de préparation des composés I selon la revendication 1 dans lesquels $R^5$ représente un groupe $CO_2H$, caractérisé par le fait que l'on hydrolyse de manière connue en soi, en présence d'une base aqueuse, un amide d'acide pyrazol-3-carboxylique (I) dans lequel $R^5$ représente un groupe $CO_2R'$ et $R^4$ alkyle en C1-C4

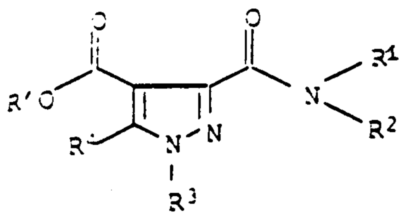

I,
($R^5$ = $CO_2R'$)

3. Procédé de préparation des composés I selon la revendication 1, dans lesquels $R^5$ représente un groupe $COYR^6$, caractérisé par le fait que l'on transforme, de manière connue en soi, un amide d'acide pyrazol-3-carboxylique (I), dans lequel $R^5$ représente un groupe $CO_2H$,

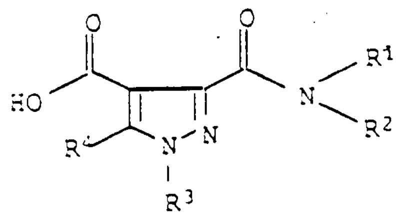

I,
($R^5$ = $CO_2H$)

en l'halogénure ou une autre forme activé de l'acide carboxylique et on estérifie ensuite ces dérivés avec un composé V

$HYR^6$ (V)

4. Agent herbicide, contenant au moins un amide d'acide pyrazol-3-carboxylique de la formule générale I selon la revendication 1 et des additifs inertes usuels.

5. Procédé de lutte contre une croissance des plantes indésirables, caractérisé par le fait qu'on traite les plantes indésirables et/ou leur biotope avec une quantité à effet herbicide d'un amide d'acide pyrazol-3-carboxylique I selon la revendication 1.